**Europäisches Patentamt**

**European Patent Office**

**Office européen des brevets**

(19)

(11) Veröffentlichungsnummer : **0 120 224**
**B1**

(12) **EUROPÄISCHE PATENTSCHRIFT**

(45) Veröffentlichungstag der Patentschrift :
15.03.89

(21) Anmeldenummer : **84101046.5**

(22) Anmeldetag : **02.02.84**

(51) Int. Cl.⁴ : **A 61 K   7/50**, A 61 K   7/48

(54) **Funktionelles Ölcremebad.**

(30) Priorität : **02.03.83 DE 3307297**

(43) Veröffentlichungstag der Anmeldung :
**03.10.84 Patentblatt 84/40**

(45) Bekanntmachung des Hinweises auf die Patenterteilung : **15.03.89 Patentblatt 89/11**

(84) Benannte Vertragsstaaten :
**AT BE CH DE FR GB IT LI NL SE**

(56) Entgegenhaltungen :
**FR–A– 1 337 769**
**FR–A– 2 293 924**
**US–A– 3 867 300**
**CHEMICAL ABSTRACTS, Band 94, Nr. 14, April 1981,**
**Seite 404,Nr.109371p, Columbus, Ohio, US; & JP - A -**
**80 162 713 (YASUNISHI, KOSAKU) 18.12.1980**

(73) Patentinhaber : **Beiersdorf Aktiengesellschaft**
**Unnastrasse 48**
**D-2000 Hamburg 20 (DE)**

(72) Erfinder : **Engel, Walter**
**Diesterwegstrasse 22**
**D-2080 Pinneberg (DE)**
Erfinder : **Hoppe, Udo, Dr. Dipl.-Chem.**
**Lottbeker Weg 7**
**D-2000 Hamburg 65 (DE)**
Erfinder : **Sauermann, Gerhard, Dr. Dipl.-Chem.**
**Hambrook 4**
**D-2351 Wiemersdorf (DE)**

EP 0 120 224 B1

**Beschreibung**

Die Erfindung betrifft ein Ölcremebad-Präparat, welches in üblicher Menge dem Badewasser zugesetzt, nicht nur einen kosmetischen Effekt ergibt, indem es die Haut weich, seidig und geschmeidig macht, wobei diese Wirkung über einen längeren Zeitraum nach der Anwendung erhalten bleibt, sondern das darüber hinaus auch einen regenerierenden und normalisierenden Effekt auf der Haut zu bewirken vermag.

Wannen und Duschbäder unter Zusatz geeigneter kosmetisch wirksamer Badezubereitungen (Badezusatzpräparate) erfreuen sich beim Verbraucher einer zunehmenden Beliebtheit, da sie nicht nur der Körperreinigung dienen, sondern beim Baden zugleich auch ein Gefühl der Sauberkeit, Entspannung und Erfrischung entstehen lassen, das für die physiologische und psychologische Wirkung des Bades auf den gesamten Organismus von erheblicher Bedeutung ist.

Zu den möglichen, äußerlich anzuwendenden, dermal wirksamen Zubereitungen gehören insbesondere Badezubereitungen auf Lipidbasis. Durch derartige Bäder können aktiv Lipide auf die Haut aufgebracht werden. Da andererseits der günstige Einfluß von Lipiden zum Schützen der Haut und zur Normalisierung des Zustandes der Haut, insbesondere trockener Haut, seit langem bekannt ist, werden Ölbäder vielfach zu dem Zweck eingesetzt, Lipide auf fettarmer Haut aufzubringen, wobei dann der Reinigungseffekt oft in den Hintergrund tritt.

Bekanntlich führt bereits das einfache Wasserbad, d. h. eines Bades ohne den Zusatz eines für diesen Zweck entwickelten Badepräparates, zu strukturellen und funktionellen Veränderungen des Hautoberflächenfilmes. Dieses Interaktionsprodukt besteht chemisch aus den Hornschichtmaterialien der Keratinisierung der Epithelzellen, aus den polaren wasserlöslichen Hautinhaltsstoffen, dem unpolaren lipidlöslichen Sebum mit seiner komplizierten Zusammensetzung und Wasser, welches sowohl frei als auch gebunden vorkommt. Es stellt ein komplexes, in sich geschlossenes, physiologisches System dar.

Schon bei einem einfachen (reinen) Wasserbad ohne jeden Zusatz kommt es zunächst zu einer Quellung der Hornschicht, die von der Dauer des Bades und seiner Temperatur abhängig ist. Gleichzeitig werden wasserlösliche Stoffe der Hautoberfläche (z. B. wasserlöslicher Schmutz), aber auch Stoffe, die für das Wasserbindungsvermögen der Hornschicht verantwortlich sind, durch Lösung entfernt. Zusätzlich kommt es durch körpereigene Emulgatoren auch noch zu einer gewissen Fettlösung. Dies bedingt nach der anfänglichen Quellung später eine deutliche Austrocknung der Haut, die durch waschaktive Zusätze noch verstärkt wird, vor allem in Richtung auf die Entfettung. Somit wird das natürliche Gleichgewicht der Inhaltsstoffe gestört und das verbleibende Material ändert seine Eigenschaften. Letztlich wird die Hydrationsfähigkeit des Hautoberflächenfilms erkennbar vermindert.

Bei intakter Haut ist diese Schädigung belanglos. Die Schutzmechanismen der Haut kompensieren derartige leichte Störungen der oberen Hautschichten ohne weiteres. Aber bereits bei Vorliegen nichtpathologischer Abweichungen, z. B. durch umweltbedingte Abnutzungsschäden bzw. Irritationen, Lichtschäden, Altershaut usw., ist der Schutzmechanismus des Hautoberflächenfilms gestört und alsdann allein oftmals nicht mehr in der Lage, seine Aufgabe zu erfüllen. Er muß dann durch externe Maßnahmen regeneriert werden.

Bei Durchsicht der veröffentlichten Rezepturen von bekannten kosmetischen Badezubereitungen ist erkennbar, daß diese zumeist aus einfachen Abmischungen von Lipiden, d. h. von Fetten, Wachsestern, höheren Alkoholen, Kohlenwasserstoffen und dgl., bestehen, die jeweils für sich allein oder in Kombination miteinander eingesetzt werden. Je nach dem vorgesehenen Anwendungszweck werden die Eigenschaften dieser Fett- oder Ölphase durch Zugabe von oberflächenaktiven Substanzen variiert, wobei diese unter dem Gesichtspunkt ausgewählt werden, daß bei einer relativ guten reinigenden Wirkung eine möglichst geringe entfettende Wirkung auftritt. Dabei können je nach Art und Menge der gewählten Bestandteile Zusammensetzungen (Badezusatzpräparate) formuliert werden, die auf der Badewasseroberfläche entweder spreitende Ölfilme, Öl-in-Wasser-Emulsionen oder auch Totalsolubilisate ergeben, wobei sowohl schäumende als auch nicht-schäumende Formulierungen möglich sind. Obwohl mit den bekannten Badezusatzpräparaten im Hinblick auf deren relativ einfache Zusammensetzung in vielen Fällen bereits ausgezeichnete Hauteffekte erzielt werden, die durch biophysikalische Vermessung der Hautparameter noch objektiviert werden können, beschränkt sich die Funktionalität derartiger Formulierungen bei Öl- oder Ölcremebad-Präparaten bis heute nur auf die Rück-, Auf- oder Überfettung der obersten Hautschichten.

Versuche, funktionelle Ölcremebäder zu entwickeln, deren Leistung über einen hautpflegenden und rückfettenden Effekt hinausgehen, verliefen bisher in jeder Weise unbefriedigend und führten nicht zu den gewünschten Ergebnissen bei ihrer Anwendung. Hierbei darf nicht übersehen werden und ist erschwerend zu berücksichtigen, daß die übliche Anwendungskonzentration des Badezusatzpräparates in einem Bad bei einer Verdünnung Badezusatz zu Wasser : 1 : 5000 beträgt. Bei einer Badewassermenge von z. B. 100 Litern (1) ist im Härtebereich 3 (2,5-3,8 mmol/IGH) mit der chemischen Reaktivität von 15 bis 21 g Gesamthärtebildnern, berechnet als CaO, zu rechnen. Die allgemein leicht alkalische Reaktion des Wassers ist bekannt.

Aufgabe der Erfindung war die Entwicklung eines funktionellen Ölcremebades, das in üblicher Menge dem Badewasser zugesetzt, neben einem hautpflegenden und rückfettenden Effekt gleichzeitig

EP 0 120 224 B1

auch einen hautregenerierenden Effekt aufweist. Dabei wird unter dem Begriff : « Hautregenerieren » die Wiederherstellung und Rückführung der Haut in den gesunden Zustand mit ihren damit verbundenen speziellen Eigenschaften verstanden.

Gegenstand der Erfindung ist daher ein funktionelles Ölcremebad, das dadurch gekennzeichnet ist, das es neben den üblichen Bestandteilen in den für solche Mittel üblichen Mengen als Wirkstoff Pelargonsäure (n-Nonylsäure) enthält.

Dabei kann die Menge der in dem Ölcremebad eingesetzten Pelargonsäure 0,5 bis 70 Gewichts-%, bezogen auf die Gesamtzusammensetzung des Präparates betragen, zweckmäßig jedoch 0,5 bis 11 Gewichts-% und vorzugsweise 2 bis 5 Gewichts-%.

Bedingt durch ihren besonderen Status, den biologischen Daten sowie ihren physikalischen und chemischen Eigenschaften vermag unter den ähnlich aufgebauten Verbindungen allein die Pelargonsäure alle Bedingungen zu erfüllen, die an die Eignung die spezielle erfindungsgemäße Verwendung gestellt werden. Dies konnte anhand entsprechender Vergleichsversuche ermittelt werden.

Die Verwendung von Pelargonsäure bzw. deren Derivaten in kosmetischen Zusammensetzungen ist an sich bekannt. So beschreibt FR-A-1 337 769 Parfümstabilisatoren mit einem Gehalt von 1-10 Gewichts-% an Pelargonsäure bzw. Pelargonsäuresalzen. FR-A-2 293 924 beschreibt den antimikrobiellen Effekt gewisser Pelargonester. Die antimikrobielle Wirkung von Fettsäuren mit 8-11 Kohlenstoffatomen wird in US-A-3, 867, 300 offenbart. JP Kökai Tokkyo Koho 80/162. 713 beschreibt bakterizide pastöse Zusammensetzungen.

Der Einsatz von Säuren oder sauren Puffergemischen zur Beeinflussung von Hautparametern war ebenfalls bekannt. Unter den Bedingungen eines Wasserbades aber war die Verwendung von Säuren (mit Ausnahme von Kohlensäure-Bädern) aus den verschiedensten Gründen mit dermatologischen oder toxikologischen Nachteilen verbunden. Demgegenüber vermeidet die Verwendung von Pelargonsäure in Ölcremebädern diese Nachteile weitestgehend, da bei dieser speziellen Verwendung die Kombination wertvoller Eigenschaften vorteilhaft zum Tragen kommt, wie die geringe, aber meßbare Löslichkeit der Pelargonsäure in Wasser ; ihre sehr gute, bevorzugte Löslichkeit in Lipiden ; das geringe, aber für den Anwendungszweck ausreichende chemische Reaktionsvermögen ; der Lipidcharakter ; der im physiologischen Bereich liegende pH-Wert der konzentrierten wässrigen Lösung ; ihre antimikrobiellen Eigenschaften ; das mit den Härtebildnern des Wasser eventuell entstehende Pelargonatgemisch mit seinen kosmetischen Eigenschaften ; die ausreichende Fähigkeit zur Protonenlieferung zur Stabilisierung des Haut-pH-Wertes sowie die Nichtbeeinflussung des pH-Wertes des Badewassers.

Entscheidend für das Wirksamwerden ist aber vor allem die Verteilungszahl zwischen (Pelargonsäure in Öl) : Wasser. Dies bedeutet für die Praxis : Es muß die ausgezeichnete und bevorzugte Löslichkeit der Pelargonsäure in der Lipidphase der Badezubereitung bei geringster Auswanderung in die Wasserphase durch entsprechende Formulierung sichergestellt werden. Derartige Bäder vermögen eine geregelte Freisetzung einer oder mehrerer kosmetisch wirksamen Substanzen zu bewirken.

Die Ölcremebad-Präparate gemäß der Erfindung enthalten 75 bis 90 % öligen Materials (Lipide), vorzugsweise in Form von pflanzlichen Ölen wie Sojaöl, Sesamöl, Erdnußöl, Sonnenblumenöl, Baumwollsaatöl und Rizinusöl, die einzeln oder im Gemisch miteinander verwendet werden können. Die genannten Pflanzenöle können ganz oder teilweise durch Mineralöle (pharmaz. Weißöle hoher Qualität wie Paraffin- und Vaselineöle) ersetzt werden. Sie können weiterhin in geringen Mengen als Rückfettungsmittel und Lösungsvermittler bzw. Erweichungsmittel natürliche oder synthetische Ester wie Isopropylmyristat, Isopropylpalmitat, Ölsäuredecylester oder auch 2-Octyldodecanol enthalten, außerdem Lanolin- und Cholesterinderivate sowie Parfumöle und andere nicht wasserlösliche Substanzen, die antiseptische, bakterizide oder bakteriostatische Eigenschaften aufweisen in den für solche Präparate üblichen Mengen.

Vorzugsweise sollen die erfindungsgemäßen Ölcremebad-Präparate bis 12 Gewichts-%, bezogen auf die Gesamtzusammensetzung, eines Polyäthylenglykolmono- oder -diesters oder eines anderen Emulgators enthalten. Dabei hat sich der Einsatz von Poläthylenglykol (400)-dioleat als besonders zweckmäßig erwiesen. Weiterhin können sie als zusätzliche oberflächenaktive Mittel Alkyläthersulfate, wie z. B. aminneutralisiertes Lauryläthersulfat, enthalten, die in Form ihrer Salze eingesetzt, selbst in hoher Verdünnung und in hartem Gebrauchswasser ein gutes Schaumvermögen zeigen und sich durch ein ausgezeichnetes Fett- und Kalkseifendispergierungsvermögen auszeichnen. Die Auswahl der Bestandteile, die sämtlich nicht toxisch und nicht reizend sein dürfen, hat unter dem Gesichtspunkt zu erfolgen, daß der ölige Anteil möglichst hoch, der Anteil an Emulgator bzw. oberflächenaktiven Substanzen dagegen möglichst niedrig liegen muß. Darüber hinaus muß eine weitgehende Stabilität der Viskosität des Ölcremebades über einen möglichst großen Temperatur-bereich sichergestellt werden.

Bedingt durch den relativ hohen Anteil des Ölcremebades gemäß der Erfindung an pflanzlichen Ölen bedarf es des Zusatzes eines Konservierungs- bzw. Antioxydationsmittels in der für derartige Zusammensetzungen üblichen Menge, wobei berücksichtigt werden muß, daß diese Menge ausreichen muß, um auch einen mikrobiellen Verderb des Produktes während der Lagerung, des Transports und beim Verbraucher zu verhindern. Für diesen Zweck können beispielsweise Benzoate, Benzoesäure-Derivate und Sorbate, sowie Verbindungen wie 2,6-Di-tert.-butyl-4-methylphenol (« Ionol ») oder 5-Brom-5-nitro-1,3-dioxan eingesetzt werden. Darüber hinaus kann des Ölcremebad auch Farbstoffe und Verdickungsmittel enthalten.

3

Die jeweils einzusetzende Menge an Parfüm und gegebenenfalls an Farbstoffen und Verdickungsmitteln kann in Abhängigkeit von der speziellen Zusammensetzung des Produktes vom Fachmann durch einfaches Ausprobieren leicht ermittelt werden.

Die Herstellung des erfindungsgemäßen Ölcremebades erfolgt in üblicher Weise durch einfaches Vermischen der Bestandteile unter leichtem Rühren. Sie bietet keine Schwierigkeiten.

Zum Nachweis der Überlegenheit des erfindungsgemäßen Ölcremebades mit Pelargonsäure gegenüber einem bekannten identisch zusammengesetzten Ölbad ohne diesen Zusatz wurden Versuche durchgeführt, bei denen die Beeinflussung des pH-Wertes der Hautoberfläche durch Bademaßnahmen und ihre Regulierung durch Öl-bzw. Ölcremebadzusätze gemessen wurde. Zu diesem Zweck wurden aus einem Gesamtkollektiv von 20 Probandinnen jene ausgewählt, die insbesondere zur Zielgruppe der « Ölbadbedürftigen » gehören. Diese ausgewählten Probandinnen mit eher lipidarmer, trockener Haut hatten ein Durchschnittsalter von 48 Jahren (s = 5,7 a ; N = 10) und einen mittleren Hautlipidgehalt von E = 0,227 (s = 0,095 : N = 56) (Der Wert für ausgesprochen trockene Haut beträgt : E $\geqslant$ 0,25). Es zeigte sich, daß direkt nach dem Stadtwasser-Bade die Azidität der Hautoberfläche an den Testarealen in hochsignifikanter Weise (mit 99,9 %iger Sicherheit, Paarvergleiche) von im Mittel pH 5,24 auf Werte zwischen pH 5,8 und 6,0 anstieg. Diese relative Alkalisierung der Haut wird in einer Gegenreaktion der Haut im Verlauf von mehreren Stunden in Richtung Ausgangswert gesteuert (rückreguliert). Bei Zusatz des Ölbades ohne Pelargonsäure-Gehalt und des erfindungsgemäßen Ölcremebades mit Pelargonsäure-Gehalt (2,5 %, bezogen auf die Gesamtzusammensetzung) in jeweils der gleichen Menge zu den Stadtwasser-Bädern konnte festgestellt werden, daß die Haut-pH-Wertregulation 45 Minuten nach Verlassen des Bades im Falle des Ölcremebades mit Pelargonsäure-Gehalt deutlich signifikant besser ist als beim reinen Wasserbad und dem mit dem bekannten Ölbad versetzten Wasserbad. Dabei wurden als Halbwertzeit für die Rückregulierung des pH-Wertes auf den Ausgangswert beim reinen Stadtwasser-Bad ein Wert von über 3 Stunden, beim Stadtwasser-Bad mit herkömmlichem Ölbadzusatz ein Wert von etwe 50 Minuten und beim Stadtwasser-Bad mit dem Zusatz des erfindungsgemäßen Pelargonsäure-haltigen Ölcremebades ein solcher von lediglich 20 Minuten ermittelt. Aus diesem Versuchsergebnis die ist Überlegenheit des erfindungsgemäßen Ölcremebades über die herkömmlichen bekannten Ölbäder eindeutig ersichtlich.

## Beispiel 1

|  | Gew.-% |
|---|---|
| Sojaöl | 38,75 |
| Rizinusöl | 2,00 |
| Vaselineöl | 37,00 |
| Pelargonsäure | 2,50 |
| 2-Octyldodecanol | 2,00 |
| Polyäthylenglykol (400)-dioleat | 10,00 |
| Hilfsstoffe, Farbstoffe, Parfum (Stabilisatoren) | ad 100,00 |

## Beispiel 2

|  | Gew.-% |
|---|---|
| Nußöl | 40,00 |
| Paraffinöl | 40,00 |
| Pelagonsäure | 5,00 |
| Polyäthylenglykol (400)-dioleat | 12,00 |
| Hilfsstoffe, Stabilisatoren, Parfum | ad 100,00 |

## Patentansprüche

1. Funktionelles Ölcremebad, dadurch gekennzeichnet, daß es neben den üblichen kosmetischen Bestandteilen
Pelargonsäure als Wirkstoff
eine Ölphasenkomponente
einen Emulgator enthält.

2. Funktionelles Ölcremebad nach Anspruch 1, dadurch gekennzeichnet, daß es Pelargonsäure in einer Menge von 0,5 bis 70 Gewichts-%, vorzugsweise in einer solchen von 0,5-11 Gewichts-%, besonders vorteilhaft in einer solchen von 2-5 Gewichts-%, bezogen auf die Gesamtzusammensetzung des Präparates, enthält.

3. Funktionelles Ölcremebad nach den Ansprüchen 1 oder 2, dadurch gekennzeichnet, daß es 75-90 Gew.-% einer Ölphasenkomponente enthält.

4. Funktionelles Ölcremebad nach Anspruch 1, 2 oder 3, dadurch gekennzeichnet, daß der Gehalt an

4

dem Emulgator 0,1-12 Gewichts-%, bezogen auf die Gesamtzusammensetzung des Präparates, beträgt.

5. Funktionelles Ölcremebad nach einen oder mehreren der Ansprüche 1-4, dadurch gekennzeichnet, daß der Emulgator aus der Gruppe der Polyethylenglycolmono- und -diester gewählt wird.

6. Funktionelles Ölcremebad nach einen oder mehreren der Ansprüche 1-5, dadurch gekennzeichnet, daß die Ölphasenkomponente gewählt wird aus der Gruppe

Sojaöl, Sesamöl, Erdnußöl, Sonnenblumenöl, Baumwollsaatöl, Rizinusöl

Paraffinöl, Vaselineöl

Lanolinöl, Cholesterinderivate

Isopropylmyristat, Isopropylpalmitat

Parfümöl

## Claims

1. Functional oil cream bath, characterized in that it contains, besides the customary cosmetic constituents,

pelargonic acid as active substance

an oil phase component

an emulsifier.

2. Functional oil cream bath according to Claim 1, characterized in that it contains pelargonic acid in an amount of 0.5 to 70 % by weight, preferably in an amount of 0,5-11 % weight, particularly advantageously in an amount of 2-5 % by weight, based on the total composition of the product.

3. Functional oil cream bath according to Claims 1 or 2, characterized in that it contains 75-90 % by weight of an oil phase component.

4. Functional oil cream bath according to Claim 1, 2 or 3, characterized in that the content of emulsifier is 0.1-12 % by weight based on the total composition of the product.

5. Functional oil cream bath according to one or more of Claims 1-4, characterized in that the emulsifier is chosen from the group of polyethylene glycol monoesters and diesters.

6. Functional oil cream bath according to one or more of Claims 1-5, characterized in that the oil phase component is chosen from the group comprising

soya oil, sesame oil, arachis oil, sunflower oil, cottonseed oil, castor oil

liquid paraffin, liquid petrolatum

lanolin oil, cholesterol derivatives

isopropyl myristate, isopropyl palmitate

perfume oil.

## Revendications

1. Bain de crème huileux fonctionnel, caractérisé en ce qu'il contient en plus des constituants cosmétiques habituels

de l'acide pélargonique comme principe actif

un composant de phase huileuse

un émulsionnant.

2. Bain de crème huileux fonctionnel suivant la revendication 1, caractérisé en ce qu'il contient l'acide pélargonique en une quantité de 0,5 à 70 % en poids, de préférence en une quantité de 0,5 à 11 % en poids et avec une préférence particulière en une quantité de 2 à 5 % en poids, sur base de la composition totale de la préparation.

3. Bain de crème huileux fonctionnel suivant la revendication 1 ou 2, caractérisé en ce qu'il contient 75 à 90 % en poids d'un composant de phase huileuse.

4. Bain de crème huileux fonctionnel suivant la revendication 1, 2 ou 3, caractérisé en ce que la teneur en émulsionnant est de 0,1 à 12 % en poids, sur base de la composition totale de la préparation.

5. Bain de crème huileux fonctionnel suivant une ou plusieurs des revendications 1 à 4, caractérisé en ce que l'émulsionnant est choisi dans la classe des mono- et diesters de polyéthylèneglycol.

6. Bain de crème huileux fonctionnel suivant une ou plusieurs des revendications 1 à 5, caractérisé en ce que le composant de phase huileuse est choisi dans la classe formée par

l'huile de soya, l'huile de sésame, l'huile d'arachide, l'huile de tournesol, l'huile de coton, l'huile de ricin

l'huile de paraffine, l'huile de vaseline

l'huile de lanoline, les dérivés de cholestérol

le myristate d'isopropyle, le palmitate d'isopropyle

une huile essentielle.